# EUROPEAN PATENT SPECIFICATION

(11) **EP 2 674 139 B1**
(45) Date of publication and mention of the grant of the patent: **25.02.2015**
(21) Application number: 12172176.5
(22) Date of filing: 15.06.2012
(51) Int. Cl.: A61F 13/62, A61F 13/15, A61F 13/56

(54) **Continuous closure tape roll for a diaper with precoated inactive adhesive regions**
Fortlaufende Verschlussklebebandrolle für eine Windel mit vorbeschichteten inaktiven Klebebereichen
Rouleau de ruban de fermeture continue pour couche avec régions adhésives inactives pré-revêtues

(43) Date of publication of application: 18.12.2013
(73) Proprietor: Nitto Bento Bantcilik San. Tic. A.S., 34510 Esenyurt Istanbul (TR)
(72) Inventor: Kus, Ercan, 34909 Istanbul (TR)
(74) Representative: Sevinç, Erkan

(56) References cited:
- EP-A2- 0 826 354
- WO-A1-96/21413
- WO-A1-2008/150819
- US-A1- 2007 265 592

## Description

### Technical Field of the Invention

The invention relates to an all-in-one manufactured easily attachable closure tape for diapers. The closure tape of the present invention comprises a mechanical fastener portion along with an adhesive portion for fastening the closure tape to a suitable location of a diaper for effecting closure of the diaper around the waist of the wearer wherein said closure tape can directly be incorporated by adhesives into a diaper body without requiring additional processing steps

### Background of the Invention

The present invention generally relates to diapers the closure tapes of which can adhesively be incorporated into a diaper body without requiring additional processing steps.

There have been a wide variety of products in the market for fastening diapers. These have been generally in the form of pressure sensitive adhesive tapes and more recently mechanical fastening systems comprising a hook and a loop element.

Mechanical fastening systems rely on a hook affixed to a closure tape, in turn affixed onto the ear or side of a diaper. The loop material is usually in the form of a frontal tape which is also designated as a landing zone. When hook material is pressed against the loop material, the resulting bond is satisfactory in terms of its shear and peel strength and closure of the diaper around the abdomen of the wearer can be securely achieved.

A mechanical fastener system may comprise a hook strip affixed to the underside of a fastening tab. When the fastening tab is lifted from the side or ear of a diaper in order to close the diaper around a wearer's abdomen, its hook grips onto the (frontal tape) nonwoven (or the like) substrate placed on the front surface of the diaper.

Diaper manufacturers utilize closure tapes with or without release tapes. In the former case because of the low strength of the pressure sensitive adhesive coated on the fastener tape, another release tape has to be laminated with pressure sensitive adhesive on one side and silicone coated on the other side to stick on the diaper from both sides, thus increasing the material cost. In the latter case a further processing station in the machinery is mandatory as adhesive must be applied to the closure tapes for effecting coupling to the diaper bodies, i.e. ears. This both increase overall costs in terms of material and man/hour and requires fine tuning of the machinery as the limited open time of the adhesives generally necessitates a strictly standardized processing routine. Since the time-dependency of the adhesives' active state is crucial and any deviation from predefined processing routine will result in extensive time and material loss due to said adhesives' inactive state being reached, a more controllable processing step is preferred by manufacturers.

To that end, diaper manufacturers acquiring closure tapes with release tapes with high material costs from third parties in the form of rolls, attach the closure tapes to the diaper bodies or in the event rolls with no release tapes are purchased, which poses a more convenient and less complex option in terms of manufacturing processes, apply adhesive to the closure tapes subsequent to unwinding the rolls and attach said tapes to the diaper bodies within the limited open time of the adhesives.

The present invention overcomes the problems delineated above by shortening machine processing routines such that no additional steps for application of adhesives are required. Therefore, a diaper manufacturer acquiring closure tapes in form of rolls with no release tapes will be able to directly attach the tapes onto diaper bodies without adhesive application step, only with a simple heated cylinder to activate the pre-glued closure tape without release.

### Objects of the Invention

An object of the present invention is to provide a reliable closure system easily and directly attachable to a diaper body for use with diapers, which closure system comprises a mechanical fastening strip that enables the user to repeatedly and effectively re-open and close the incontinence garment to which the closure system is attached.

Another object of the present invention is to provide a reliable closure system for use with diapers necessitating no additional manufacturing process steps in the machinery for applying adhesive to said closure system.

### Summary of the Invention

The present invention provides a closure system with a fastening tab incorporating a hook portion into an ear section that comes into contact with a frontal tape (or loop portion) in order to close the diaper around the wearer body.

Said closure tape comprises a mechanical fastener strip comprising said hook portion which extends in the machine direction. Said closure tape preferably comprises a non-woven base with an adhesive applied surface to which said mechanical fastener strip.

The multi-layered closure system according to the present invention is produced, stored and supplied on a spool or roll which carries a certain length of closure tape and is designed for continuous feed into a diaper manufacturing line. The required length of tape is then cut at right angles to the spool winding direction by the diaper manufacturing line and is affixed onto the ears or sides of the diaper.

While the multi-layered closure system of the present invention is affixed to its locations on the ears of a diaper, a pre-coated adhesive region of the closure tape is activated by heat. To effect this, a certain length of the closure system according to the invention in the form of a roll or spool is continuously fed into a manufacturing line where said pre-coated adhesive regions are activated on heated cylinders to be subsequently cut to specific shapes in the form of closure tapes directly attachable to said diaper bodies.

### Brief Description of Figures

The figure whose brief explanation is herewith provided is solely intended for providing a better understanding of the present invention and is as such not intended to define the scope of protection or the context in which said scope is interpreted in the absence of the description.
Fig. 1 demonstrates a schematic view of a closure tape for a diaper according to the present invention.
Fig. 2 demonstrates process steps associated with the present invention in the form of work flow.

### Detailed Description of the Invention

The present invention provides a closure system with a closure tape (11) having a non-woven base (15) into which a strip of mechanical fastener (13) is adhered.

The multi-layered closure system according to the present invention is produced, stored and supplied on a spool or roll which carries a certain length of tape and is designed for continuous feed into a diaper manufacturing line. The required length of tape is then cut at right angles to the spool winding direction by the diaper manufacturing line and is affixed onto the sides of the diaper.

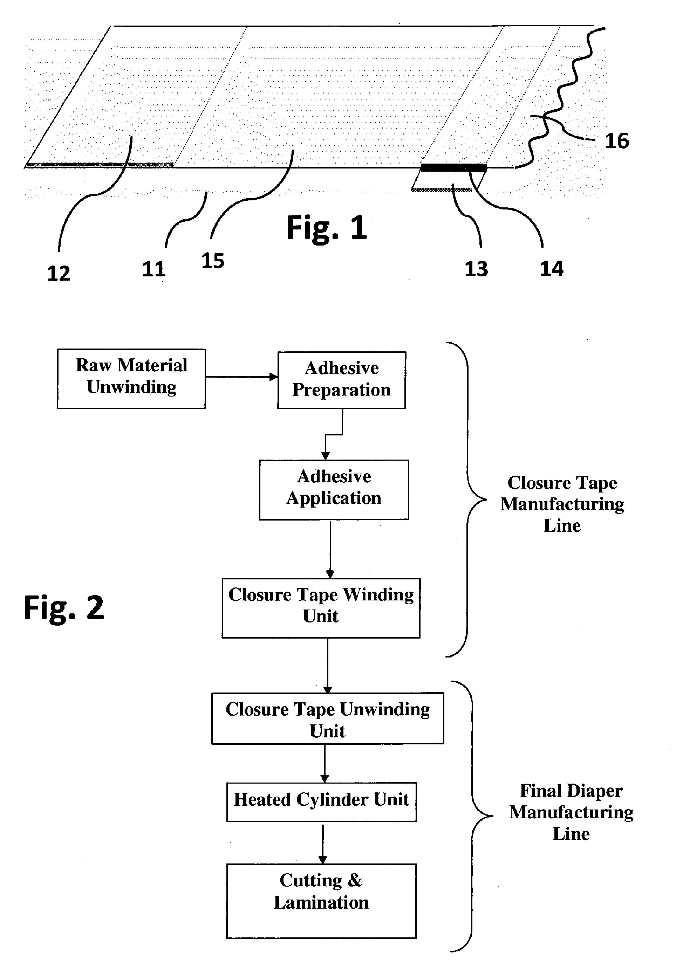

The closure system in the form of a diaper fastening tape according to the present invention provides a construction of a continuous strip as shown in Fig. 1, which is adapted to be joined to a diaper chassis after being cut in the form of individual fastening tapes (11), each having a mechanical fastener strip (13).

While said mechanical fastener strips (13) run through the length of said continuous strip in the machine direction, a first distal longitudinal edge of said fastening tape (11) is designed as a fingerlift (16) and a second distal longitudinal edge (12) thereof provides diaper chassis connection.

Said closure tape (11) preferably comprises a non-woven base (15) with an adhesive applied surface (14) to which said mechanical fastener strip (13) is joined. The mechanical fastener strips (13) being designed in the form of hook portions are adherable to a textile frontal tape.

The micro hooks have extensive gripping properties and grip most surfaces to a certain degree, except thermoplastic derivatives such as polyethylene and polypropylene or co-extruded films. Optimum gripping properties are achieved when the hook is pressed against a loop material that provide in optimum sheer and peel values. These typically range at least 30 N for sheer values (astmd 5170-91) and at least 1.5 N for peel values (astmd 5170-91) when tested on zwick/ roell z 2.5 tester.

While the multi-layered closure system of the present invention is affixed to its locations on the ears of a diaper, a pre-coated adhesive region (12) of the closure tape (11) is activated by heat. The pre-coated adhesive region (12) is normally inactive until heated. Therefore, pre-coated adhesive regions (12) in a roll or spool of a closure tape (11) according to the invention do not show any adhesive character until unwound and heated. Winding into a roll or spool takes place after the open time of the adhesive is terminated. To effect activation, a certain length of the closure system in the roll or spool is continuously fed into a manufacturing line where said pre-coated adhesive regions (12) are activated on heated cylinders to be subsequently cut to specific shapes in the form of closure tapes (11) directly attachable to said diaper bodies.

In a nutshell, the present invention proposes a closure system for effecting closure of a diaper around the abdomen of a wearer wherein said closure system is provided as a continuous non-woven closure tape (11). Said continuous closure tape (11) comprises a plurality of mechanical fastener strips (13) and a plurality of adhesive fastener portions (12), each fastener and adhesive portion (13, 12) paralelly extending along the length of said continuous strip (20) in the machine direction.

According to a preferred embodiment of the present invention, a method for forming a roll which carries a certain length of closure tape suitable for continuous feed into a diaper manufacturing line is disclosed. The method entails lamination of a continuous, preferably non-woven substrate (15) with a continuous mechanical fastener strip (13) at a distal portion of said substrate (15) in the cross direction, application of adhesive to a second distal edge portion (12) of said substrate (15) in the cross direction, winding said adhesive applied continuous substrate (15) into a roll. Further, a method for attaching closures tapes to a diaper body is disclosed. The method entails unwinding a roll of a certain length of closure tape, subjecting said certain length of unwound closure tape to heat by means of heated cylinders, cutting said certain length of unwound closure tape to specific shapes in the form of closure tapes (11), attaching said shaped closure tapes (11) to diaper bodies at said adhesive pre-coated regions (12).

## Claims

1. A closure system for effecting closure of a diaper around the abdomen of a wearer, said closure system being provided as a continuous closure tape (11) and being formed into a roll, said tape (11) comprising a plurality of mechanical fastener strips (13) and a plurality of adhesive portions (12), each fastener and adhesive portion (13, 12) paralelly extending along the length of said continuous strip (20) in the machine direction **characterized in that** said continuous closure tape (11) is formed into a roll after an open time associated with said adhesive is terminated.

2. A closure system for effecting closure of a diaper around the abdomen of a wearer as set forth in Claim 1 wherein said closure system comprises a substrate (15) made out of a non-woven material.

3. A closure system for effecting closure of a diaper around the abdomen of a wearer as set forth in Claim 1 or 2 wherein said non-woven substrate (11) are silicone coated and UV cured.

4. A method for forming a roll carrying a certain length of closure tape for continuous feed into a diaper manufacturing line, said method comprising the steps of lamination of a continuous substrate (15) with a continuous mechanical fastener strip (13) at a distal portion of said substrate (15) in the cross direction, application of adhesive to a second distal edge portion (12) of said substrate (15) in the cross direction and winding said adhesive applied continuous substrate (15) into a roll after an open time associated with said adhesive is terminated.

5. A method for attaching closures tapes to a diaper body manufactured according to the method of Claim 4, said method comprising unwinding a roll of a certain length of closure tape, subjecting said certain length of unwound closure tape to heat by means of heated cylinders to activate the pre-coated adhesive, cutting said certain length of unwound closure tape to specific shapes in the form of closure tapes (11), attaching said shaped closure tapes (11) to diaper bodies at said adhesive pre-coated regions (12).

6. A method as set forth in Claim 4 wherein said substrate (15) is made out of a non-woven material.

## Patentansprüche

1. Verschlusssystem zum Verschließen einer Windel um das Abdomen eines Trägers, wobei das Verschlusssystem als kontinuierliches Verschlussband (11) bereitgestellt und zu einer Rolle geformt ist, wobei das Band (11) eine Vielzahl von mechanischen Befestigungsstreifen (13) und eine Vielzahl von Klebstoffabschnitten (12) aufweist, wobei sich jeder Befestigungs- und Klebstoffabschnitt (13, 12) parallel entlang der Länge des kontinuierlichen Streifens (20) in der Maschinenrichtung erstreckt, **dadurch gekennzeichnet, dass** das kontinuierliche Verschlussband (11) zu einer Rolle geformt wird, nachdem eine dem Klebstoff zugeordnete klebeoffene Zeit verstrichen ist.

2. Verschlusssystem zum Verschließen einer Windel um das Abdomen eines Trägers gemäß Anspruch 1, wobei das Verschlusssystem ein Substrat (15) aus einem nichtgewebten Material umfasst.

3. Verschlusssystem zum Verschließen einer Windel um das Abdomen eines Trägers gemäß Anspruch 1 oder 2, wobei das nichtgewebte Substrat (11) siliconbeschichtet und UV-gehärtet ist.

4. Verfahren zum Bilden einer Rolle, die eine gewisse Länge von Verschlussband zum kontinuierlichen Zuführen zu einer Windelherstellungsanlage trägt, wobei das Verfahren die Schritte des Laminierens eines kontinuierlichen Substrats (15) mit einem kontinuierlichen mechanischen Befestigungsstreifen (13) an einem distalen Abschnitt des Substrats (15) in der Querrichtung, des Aufbringens von Klebstoff auf einen zweiten distalen Randabschnitt (12) des Substrats (15) in der Querrichtung und des Aufwickelns des kontinuierlichen Substrats (15) mit aufgebrachtem Klebstoff zu einer Rolle, nachdem eine dem Klebstoff zugeordnete klebeoffene Zeit verstrichen ist, umfasst.

5. Verfahren zum Befestigen von Verschlussbändern an einen Windelkörper, hergestellt nach dem Verfahren gemäß Anspruch 4, wobei das Verfahren das Abwickeln einer bestimmten Länge von Verschlussband von einer Rolle, Aussetzen der bestimmten Länge von abgewickeltem Verschlussband an Wärme mithilfe geheizter Zylinder, um den vorbeschichteten Klebstoff zu aktivieren, Schneiden der bestimmten Länge von abgewickeltem Verschlussband zu spezifischen Formen in der Form von Verschlussbändern (11), Befestigen der geformten Verschlussbänder (11) an Windelkörpern an den mit Klebstoff vorbeschichteten Bereichen (12), umfasst.

6. Verfahren gemäß Anspruch 4, wobei das Substrat (15) aus einem nichtgewebten Material besteht.

## Revendications

1. Système de fermeture permettant de fermer une couche autour de l'abdomen d'un utilisateur, ledit système étant fourni comme ruban de fermeture continu (11) et formé en rouleau, ledit ruban (11) comprenant une pluralité de bandes de fixation (13) et une pluralité de zones adhésives (12), chaque bande de fixation et chaque zone adhésive (13, 12) s'étendant parallèlement, les unes aux autres, le long de la bande (20) dans le sens machine, **caractérisé en ce que** ledit ruban de fermeture continu (11) est formé en rouleau une fois qu'un temps d'ouverture associé audit adhésif est écoulé.

2. Système de fermeture permettant de fermer une couche autour de l'abdomen d'un utilisateur suivant la revendication 1, ledit système de fermeture comprenant un substrat (15) réalisé à partir d'un matériau non tissé.

3. Système de fermeture permettant de fermer une couche autour de l'abdomen d'un utilisateur suivant la revendication 1 ou 2, dans lequel lesdits substrats non-tissés (11) sont enduits de silicone et traités sous UV.

4. Procédé de formation d'un rouleau d'une certaine longueur de ruban de fermeture pour une alimentation continue d'une ligne de fabrication de couches, ledit procédé comprenant les étapes consistant à laminer, dans le sens transversal, un substrat continu (15) avec une bande de fixation mécanique continue (13) à une zone distale dudit substrat (15), à appliquer, dans le sens transversal, de l'adhésif sur une seconde zone de bord distale du substrat (15) et à enrouler ledit substrat continu, sur lequel de l'adhésif a été appliqué, en rouleau, une fois qu'un temps d'ouverture associé audit adhésif est écoulé.

5. Procédé de fixation de rubans de fermetures à un corps de couche fabriqué suivant le procédé de la revendication 4, ledit procédé comprenant le déroulement d'un rouleau d'une certaine longueur de ruban de fermeture, l'exposition de ladite certaine longueur de ruban de fermeture déroulée à la chaleur, au moyen de cylindres chauffés, pour activer l'adhésif préalablement appliqué, la découpe de ladite certaine longueur de ruban de fermeture déroulée de façon à obtenir des formes spécifiques constituant des rubans de fermeture (11), la fixation desdits rubans de fermeture formés (11) aux corps de couches au niveau desdites zones pré-enduites d'adhésif (12).

6. Procédé suivant la revendication 4, dans lequel ledit substrat (15) est réalisé à partir d'un matériau non-tissé.
